# EUROPÄISCHE PATENTSCHRIFT

(11) **EP 1 758 513 B1**
(45) Veröffentlichungstag und Bekanntmachung des Hinweises auf die Patenterteilung: **17.12.2008**
(21) Anmeldenummer: 04737130.7
(22) Anmeldetag: 23.06.2004
(51) Int. Cl.: A61B 17/72

(54) **CHIRURGISCHER NAGEL**
SURGICAL NAIL
CLOU CHIRURGICAL

(43) Veröffentlichungstag der Anmeldung: 07.03.2007
(73) Patentinhaber: Synthes GmbH, 4436 Oberdorf (CH)
(72) Erfinder: SCHLIENGER, André, CH-4144 Arlesheim (CH); BUETTLER, Markus, CH-4702 Oensingen (CH); SENN, Peter, CH-4437 Waldenburg (CH)
(74) Vertreter: Lusuardi, Werther
(86) Internationale Anmeldenummer: PCT/CH2004/000381
(87) Internationale Veröffentlichungsnummer: WO 2006/000108

(56) Entgegenhaltungen:
- WO-A-20/04096067
- DE-U1- 20 300 987
- US-A1- 2002 103 488
- US-A1- 2003 171 754

## Beschreibung

Die Erfindung betrifft einen chirurgischen Nagel, insbesondere einen intramedullären Marknagel, gemäss dem Oberbegriff des Patentanspruchs 1.

Die Verriegelung von Marknägeln gehört zum Stand der Technik. Die Einführung der Verriegelungsschrauben oder Verriegelungsbolzen (im folgenden wird nur noch der Begriff Verriegelungsschraube verwendet, der aber auch den Begriff Verriegelungsbolzen mitumfassen soll) in die Querbohrungen eines Marknagels erfolgt entweder mit Hilfe eines bildgebenden Verfahrens (Röntgenkontrolle) oder einer mehr oder weniger komplizierten Zielvorrichtung. In beiden Fällen ist eine gewisse Zielungenauigkeit nicht zu vermeiden, d.h. die Schraubenspitze lässt sich nicht exakt koaxial zur Mittelachse der Querbohrung ausrichten, sondern weicht davon um einen gewissen Betrag ab. Damit die Verriegelungsschraube trotz dieses Zielfehlers in die Querbohrung mündet und durch diese hindurchgebracht werden kann, wird der Aussendurchmesser der Schraube relativ zum Durchmesser der Querbohrung unterdimensioniert. Bleibt die Zielungenauigkeit im Rahmen dieser Unterdimensionierung, so kann die Verriegelungsschraube trotz des Zielfehlers problemlos durch die Querbohrung geführt werden. Allerdings weist nun die Verriegelungsschraube relativ zur Querbohrung - wegen der Unterdimensionierung - ein gewisses Spiel auf.

Dieses Spiel definiert, um welchen Betrag sich die Knochenhauptfragmente, welche mittels Verriegelungsschrauben im entsprechenden Verriegelungsloch fixiert werden, relativ zum Nagel und somit aufgrund der Starrheit des Nagels auch relativ zu anderen mit demselben Nagel befestigten Knochenhauptfragmenten bewegen können. Um die Anwendbarkeit der Verriegelung für den Chirurgen zu garantieren, ist dieses Spiel zwar unumgänglich, doch ist es klinisch bei gewissen Indikationen (z.B. im Falle von metaphysären Fragmenten) unerwünscht.

Selbst Nägel mit vollem Querschnitt, welche im Verriegelungsloch ein Innengewinde aufweisen können, sind nicht spielfrei. Das Innengewinde verhindert lediglich, dass der Nagel sich axial auf der Verriegelungsschraube verschieben kann.

Aus der US 6,296,645 HOVER ET AL. ist ein hohler, intramedullärer Marknagel aus Metall bekannt, der in den als Fenster bezeichneten sich diametral gegenüberstehenden Mantelöffnungen der Querbohrung einen oder zwei Kunststoffeinsätze aufweist, durch welche die Verriegelungsschraube eingeführt werden kann. Nachteilig bei diesem bekannten Marknagel ist der Umstand, dass die fensterartigen Kunststoffeinsätze leicht eingedrückt werden können, so dass die erwünschte Funktion verloren geht. Aber selbst bei einer sehr vorsichtigen Manipulation dürften die beiden Kunststoffeinsätze beim Durchführen der Verriegelungsschraube aus ihrem "Fenster" gedrückt werden, was ebenfalls zu einem Verlust der Funktion führt.

Ein weiterer Marknagel mit einer Querbohrung und einer in der Querbohrung positionierbaren Ringnut ist aus DE 203 00 987 U1 bekannt. Dieses Dokument offenbart die Merkmale des Oberbegriffs Anspruchs 1.
Hier will die Erfindung Abhilfe schaffen. Der Erfindung liegt die Aufgabe zugrunde, einen chirurgischen Nagel, insbesondere einen intramedullären Marknagel zu schaffen, mit dem das vorhandene Spiel zwischen ihm und der Verriegelungsschraube risikolos eliminiert werden kann und eine verbesserte Haltekraft sowie ein verbesserter Führungseffekt zwischen Verriegelungsschraube und Marknagel erzielt werden kann.

Die Erfindung löst die gestellte Aufgabe mit einem chirurgischen Nagel, welcher die Merkmale des Anspruchs 1 aufweist.

Damit sind folgenden Vorteile erzielbar:
a) die Zielgenauigkeit bei der Einbringung der Verriegelungsschraube ist unbeeinträchtigt;
b) der Arzt kann noch intraoperativ wählen, ob er eine winkelstabile Verriegelung der Verriegelungsschraube vornehmen will oder nicht, wobei der Begriff "winkelstabil" eine Einschränkung gewisser Freiheitsgrade bedeutet;
c) Möglichkeit der winkelstabilen Fixierung des Knochenfragments in gewissen Richtungen für einen bestimmten Betrag der Last; und
d) Nagel und Einsatz können separat steril verpackt werden und der Chirurg kann wählen, ob er den Nagel ohne Einsatz oder mit Einsatz verwenden will. In letzterem Fall kann der Chirurg den Einsatz selbst in die Querbohrung des Nagels einführen und gegebenenfalls auch wieder entfernen. Verwendet der Chirurg den Nagel ohne Einsatz so bleibt dieser weiterhin steril verpackt für eine nächste Verwendung.

Der Einsatz kann bei einer besonderen Ausführungsform mindestens teilweise mit einem Aussengewinde versehen sein. Vorteilhafterweise verläuft der Längsschlitz durchgehend über die gesamte Länge des Einsatzes. Der Einsatz kann auch mehrere, nicht über die gesamte Länge des Einsatzes verlaufende Längsschlitze aufweisen, wodurch sich eine erhöhte Flexibilität des Einsatzes ergibt. Die Längsschlitze können auch auf dem Umfang des Einsatzes versetzt angeordnet sein oder auch axial übereinander angeordnet werden.

Der Nagel besteht vorteilhafterweise aus einem Material "M" mit der Zugfestigkeit F_{z}, der Druckfestigkeit F_{d} , der Dichte ρ₂ und dem Elastizitätsmodul E und der Einsatz aus einem Material "m", welches ein geringeres Elastizitätsmodul e < E aufweist als das Material M.

Vorzugsweise ist der Einsatz im wesentlichen kongruent zur Querbohrung des Nagels ausgebildet.

Der Einsatz kann eine zu seiner Längsachse koaxiale Bohrung aufweisen und der Längsschlitz kann mit dieser koaxialen Bohrung kommunizieren.

Das Elastizitätsmodul "e" des Einsatzes beträgt vorteilhafterweise e < 0,8 E, vorzugsweise e < 0,7 E.

Das Material "m" des Einsatzes weist vorzugsweise eine Zugfestigkeit f_{z} < F_{z} und eine Druckfestigkeit f_{d} < F_{d} auf.

Das Material "m" des Einsatzes kann aus einem biokompatiblen Kunststoff, vorzugsweise einem Polyethylen oder einem hochmolekularen Polyethylen (HMWPE) bestehen. Diese Materialien haben den Vorteil, dass kein Abbau des Kunststoffs mit unbekannten Abbauprodukten erfolgt. Der Kunststoff kann auch ein bioresorbierbares Polymer oder Copolymer, vorzugsweise ein Polylactid sein. Bei dieser Ausführung resultiert anfänglich eine spielfreie Querverriegelung des Marknagels, welche dann mit zunehmender Resorption des Polymers sukzessive wieder aufgehoben wird, so dass die Querverriegelungsschraube relativ zum Marknagel und somit auch die versorgten Knochenfragmente wieder beweglich werden. Es erfolgt somit eine Dynamisierung der Knochenfragmente nach erfolgter Frakturkonsolidierung. Das bioresorbierbare Material hat den Vorteil, dass die beim Hindurchschrauben einer Verriegelungsschraube durch den Einsatz entstehenden Späne vom Körper abgebaut werden können. Ein weiterer Vorteil besteht in der Möglichkeit eine eventuell zeitlich unterschiedliche Stärke der winkelstabilen Verriegelung der Verriegelungsschraube zu realisieren, d.h. einen graduellen Abbau der Haltekraft zu erreichen.

Die Querbohrung kann eine Kreisbohrung sein, wobei in diesem Fall das Querschnittsprofil F die maximalen Längen a = b aufweist. Sie kann aber auch als Langloch ausgebildet werden, wobei das Querschnittsprofil F die maximalen Längen a > b aufweist.

Das Material "m" des Einsatzes weist vorteilhafterweise eine geringere Dichte ρ₁ als das Material M mit der Dichte ρ₂ auf, wobei vorzugsweise ρ₁ < 0,8 ρ₂ ist.

Bei der Verwendung des Nagels als Verriegelungsnagel verfügt dieser über eine in seine Querbohrung durch den Einsatzes hindurchführbare Verriegelungsschraube oder einen Verriegelungsbolzen, deren/dessen Schaft einen Durchmesser "d" aufweist, welcher den Bedingungen a > d < b gehorcht. Die Querbohrung kann sich gegen die Oberfläche des Nagels hin erweitern, vorzugsweise in Form eines konischen Abschnittes. Dies hat den Vorteil, das ein darin eingeführter Einsatz mit einem dazu korrespondierenden konischen Abschnitt sich nicht mehr axial in Einführrichtung verschieben kann.

Für die mit dem Nagel zu verwendende Verriegelungsschraube, welche einen Schaft mit Aussengewinde umfasst, beträgt der Durchmesser "d" des Aussengewindes vorteilhafterweise a > d < b , und "d" ist vorzugsweise mindestens 5 % kleiner ist als die kleinere der beiden Dimensionen a, b.

Der Einsatz kann bei einer weiteren Ausführungsform aus einem Stift mit konisch erweitertem Kopf bestehen. Der Einsatz kann auch aus einem zentral durchbohrten Stift mit mehreren peripher angeordneten, konischen Erweiterungen bestehen, welche in entsprechende Kavitäten, respektive in der Längsbohrung des Nagels im Bereich der Querbohrung im Form eines Klickverschlusses eingreifen können.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht eines durchgängig kannulierten Marknagels mit einer Querbohrung und einen dazu passenden Einsatz sowie eine Querverriegelungsschraube;
Fig. 2 einen Längsschnitt durch den Marknagel nach Fig. 1;
Fig. 3 einen um 90° gedrehten Längsschnitt durch den Marknagel nach Fig. 1;
Fig. 4 eine vergrösserte schematische Ansicht des Profils der Querbohrung des Marknagels nach Fig. 1;
Fig. 5 einen einteiligen Einsatz mit einer Querbohrung, welche nach dem Einsetzen in etwa mit der Nagelkannulierung fluchtet;
Fig. 6 einen partiellen Längsschnitt durch einen weiteren, modifizierten Marknagel mit einem modifizierten einteiligen Einsatz;
Fig. 7 einen partiellen Längsschnitt durch einen weiteren, modifizierten Marknagel mit einem modifizierten einteiligen Einsatz;
Fig. 8 eine perspektivische Ansicht eines modifizierten Einsatzes für eine Langloch-Querbohrung; und
Fig. 9 eine perspektivische Ansicht eines modifizierten Einsatzes für eine normale, kreiszylindrische Querbohrung.

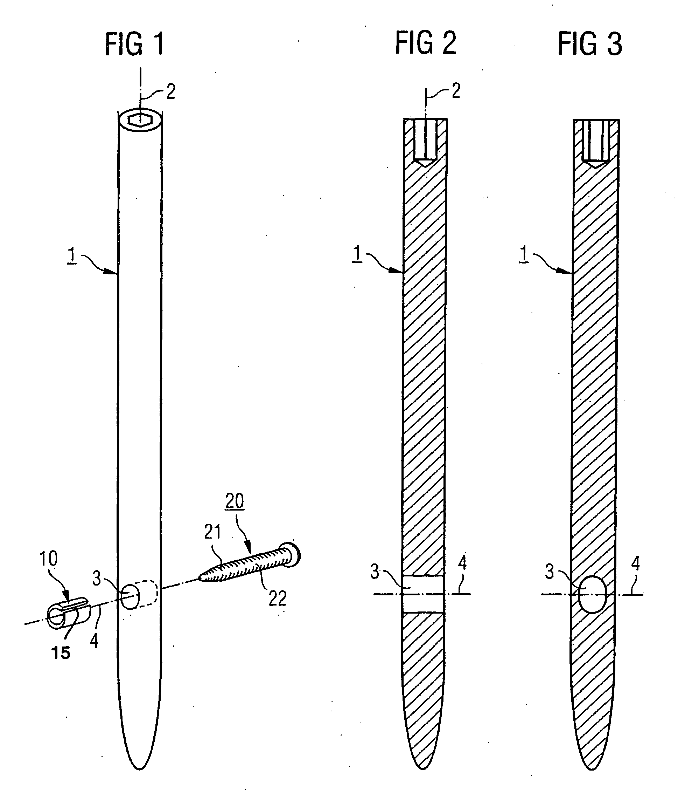

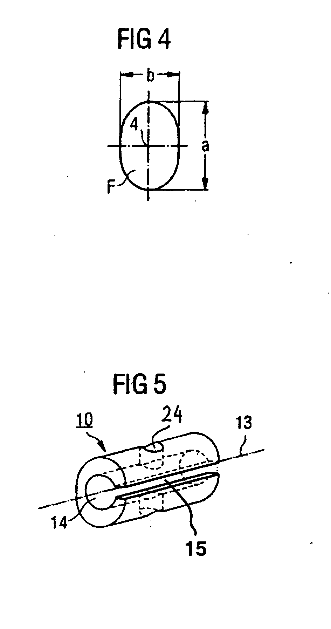

Der in den Fig. 1 - 3 dargestellte chirurgische Nagel 1 ist ein intramedullärer Marknagel für Röhrenknochen mit einer Zentralachse 2, der aus einem Metall oder einer Metalllegierung, d.h. einem Material mit relativ hoher Festigkeit (Zugfestigkeit F_{z}, Druckfestigkeit F_{d} und Elastizitätsmodul E) besteht. Der Nagel 1 besitzt eine quer zur Zentralachse 2 verlaufende, als Langloch mit dem Querschnittsprofil F ausgebildete Querbohrung 3 mit der Querachse 4. Wie in Fig. 4 dargestellt weist die Querbohrung 3 ein Querschnittsprofil F auf, welches in Richtung der Zentralachse 2 eine maximale Länge a und senkrecht dazu eine maximale Breite b aufweist. Der Nagel kann auch noch weitere - zeichnerisch nicht dargestellte - Querbohrungen 3 (rund oder oval) aufweisen.

Wie weiter in Fig. 1 dargestellt, ist ein hohler Einsatz 10 zur Einführung in die Querbohrung 3 vorgesehen, der einen durchgehenden Längsschlitz 15 aufweist. Die Dimensionierung des Einsatzes 10 ist kongruent zur Querbohrung 3, bzw. ist derart gewählt dass bei der Insertion ein Presssitz resultiert, wodurch ein Herausfallen des Einsatzes 10 aus der Querbohrung 3 verhindert wird. Der Einsatz 10 besteht aus einem Material "m" geringerer Festigkeit, insbesondere einem geringeren E-Modul (im Vergleich zum Material M des Marknagels).

Wie in Fig. 5 dargestellt weist der Einsatz 10 eine zu seiner Längsachse 13 koaxiale Längsbohrung 14 und eine nach dem Einsetzen in den Marknagel senkrecht mit dessen Kannulierung korrespondierende Querbohrung 24 auf.

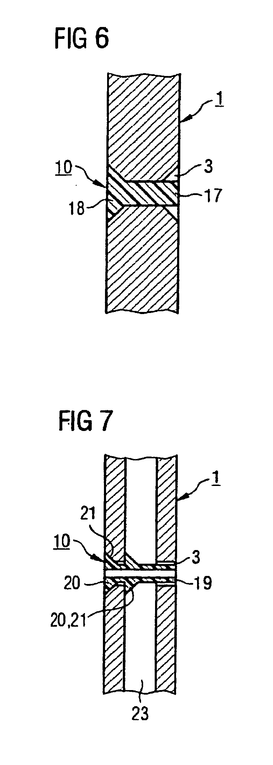

In Fig. 6 ist eine Variante eines einteiligen Einsatzes 10 dargestellt, der aus einem Stift 17 mit konischem erweitertem Kopf 18 besteht, welche in eine entsprechend ausgebildete Querbohrung 3 des Nagels 1 eingeführt werden kann.

In Fig. 7 ist eine weitere Variante eines einteiligen Einsatzes 10 dargestellt, der aus einem zentral durchbohrten Stift 19 mit mehreren peripheren angeordneten, konischen Erweiterungen 20 besteht, welche in entsprechende Kavitäten 21, respektive in der Längsbohrung 23 des Nagels 1 im Bereich der Querbohrung 3 im Form eines Klickverschlusses eingreifen können. Zu diesem Zweck ist die Querbohrung 3 des Nagels 1 geometrisch entsprechend angepasst.

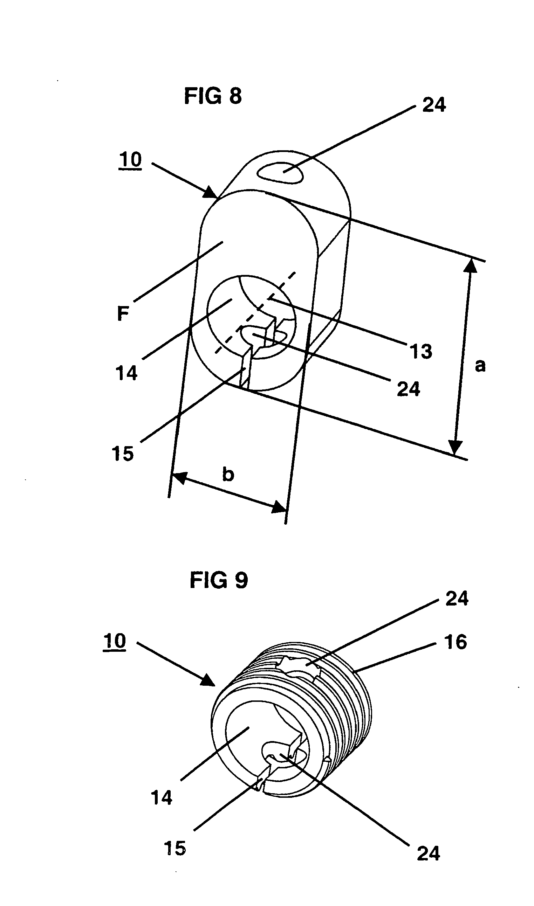

In Fig. 8 ist eine weitere Variante eines Einsatzes 10 für eine Langloch-Querbohrung dargestellt. Die Längsbohrung 14 und der parallel dazu verlaufende Längsschlitz 15 kommunizieren miteinander. Statt der dezentralen Anordnung der Längsbohrung 14 kann auch eine zentrale Anordnung gewählt werden. Analog zur Variante nach Fig. 5 weist der Einsatz 10 eine nach dem Einsetzen in den Marknagel senkrecht mit dessen Kannulierung korrespondierende Querbohrung 24 auf.

In Fig. 9 ist eine weitere Variante eines Einsatzes 10 für eine normale, kreiszylindrische Querbohrung 3 eines Nagels 1 dargestellt. Die Längsbohrung 14 und der parallel dazu verlaufende Längsschlitz 15 kommunizieren miteinander. Der Einsatz 10 trägt an seiner äusseren Mantelfläche ein Aussengewinde 16, welches vorzugsweise mit einem in der Querbohrung 3 des Nagels 1 angebrachten Innengewinde korrespondiert. Analog zur Variante nach Fig. 5 weist der Einsatz 10 eine nach dem Einsetzen in den Marknagel senkrecht mit dessen Kannulierung korrespondierende Querbohrung 24 auf.

## Patentansprüche

1. Chirurgischer Nagel (1), insbesondere intramedullärer Marknagel, mit einer Zentralachse (2) und mindestens einer quer zur Zentralachse (2) verlaufenden Querbohrung (3) mit dem Querschnittsprofil F und einer Querachse (4), wobei das Querschnittsprofil F in Richtung der Zentralachse eine maximale Länge a und senkrecht dazu eine maximale Breite b aufweist und wobei der Marknagel im Bereich der Querbohrung (3) einen Durchmesser D aufweist, und mit einem in die Querbohrung (3) einführbaren Einsatz (10) mit einer Längsachse (13) und einem Längsschlitz (15),
**dadurch gekennzeichnet, dass**
die Länge des Einsatzes (10) der Länge der Querbohrung (3) entspricht.

2. Nagel (1) nach Anspruch 1, **dadurch gekennzeichnet, dass** der Einsatz (10) mindestens teilweise mit einem Aussengewinde (16) versehen ist.

3. Nagel (1) nach Anspruch 1 oder 2, **dadurch gekennzeichnet, dass** der Längsschlitz (15) durchgehend über die gesamte Länge des Einsatzes (10) verläuft.

4. Nagel (1) nach einem der Ansprüche 1 bis 3, **dadurch gekennzeichnet, dass** der Einsatz (10) mehrere, nicht über die gesamte Länge des Einsatzes (10) verlaufende Längsschlitze (15) aufweist.

5. Nagel (1) nach Anspruch 4, **dadurch gekennzeichnet, dass** die Längsschlitze (15) auf dem Umfang des Einsatzes (10) versetzt angeordnet sind.

6. Nagel (1) nach Anspruch 4 oder 5, **dadurch gekennzeichnet, dass** die Längsschlitze - 15) axial übereinander angeordnet sind.

7. Nagel (1) nach einem der Ansprüche 1 bis 6, **dadurch gekennzeichnet, dass** er aus einem Material M mit der Zugfestigkeit F_{z} , der Druckfestigkeit F_{d}, der Dichte ρ₂ und dem Elastizitätsmodul E besteht und dass der Einsatz (10) aus einem Material m besteht, welches ein geringeres Elastizitätsmodul e < E aufweist als das Material M.

8. Nagel (1) nach einem der Ansprüche 1 bis 7, **dadurch gekennzeichnet, dass** der Einsatz (10) eine zu seiner Längsachse (13) koaxiale Bohrung (14) aufweist.

9. Nagel (1) nach Anspruch 8, **dadurch gekennzeichnet, dass** der Längsschlitz (15) mit der koaxialen Bohrung (14) kommuniziert.

10. Nagel (1) nach einem der Ansprüche 1 bis 9, **dadurch gekennzeichnet, dass** das Elastizitätsmodul "e" des Einsatzes e < 0,8 E ist.

11. Nagel (1) nach Anspruch 10, **dadurch gekennzeichnet, dass** das Elastizitätsmodul "e" des Einsatzes e < 0,7 E ist.

12. Nagel (1) nach einem der Ansprüche 1 bis 11, **dadurch gekennzeichnet, dass** die Querbohrung (3) glatt ausgebildet ist.

13. Nagel (1) nach einem der Ansprüche 1 bis 12, **dadurch gekennzeichnet, dass** die Querbohrung (3) gewindefrei ist.

14. Nagel (1) nach einem der Ansprüche 1 bis 13, **dadurch gekennzeichnet, dass** das Material m ein biokompatibler Kunststoff, vorzugsweise ein Polyethylen oder ein hochmolekulares Polyethylen (HMWPE) ist.

15. Nagel (1) nach einem der Ansprüche 1 bis 14, **dadurch gekennzeichnet, dass** der Kunststoff ein bioresorbierbares Polymer oder Copolymer, vorzugsweise ein Polylactid ist.

16. Nagel (1) nach einem der Ansprüche 1 bis 15, **dadurch gekennzeichnet, dass** die Querbohrung (3) eine Kreisbohrung ist, wobei das Querschnittsprofil F die maximalen Längen a = b aufweist.

17. Nagel (1) nach einem der Ansprüche 1 bis 15, **dadurch gekennzeichnet, dass** die Querbohrung (3) als Langloch ausgebildet ist, wobei das Querschnittsprofil F die maximalen Längen a > b aufweist.

18. Nagel (1) nach einem der Ansprüche 1 bis 17, **dadurch gekennzeichnet, dass** das Material m eine geringere Dichte ρ₁ als das Material M mit der Dichte ρ₂ aufweist, wobei vorzugsweise ρ₁ < 0,8 ρ₂ ist.

19. Nagel (1) nach einem der Ansprüche 1 bis 18, **dadurch gekennzeichnet, dass** er eine in die Querbohrung (3) durch den Einsatz (10) hindurchführbare Verriegelungsschraube (20) oder einen Verriegelungsbolzen umfasst, dessen Schaft (21) einen Durchmesser "d" aufweist, welcher den Bedingungen a > d < b gehorcht.

20. Nagel (1) nach einem der Ansprüche 1 bis 19, **dadurch gekennzeichnet, dass** sich die Querbohrung (3) gegen die Oberfläche des Nagels (1) hin erweitert, vorzugsweise in Form eines konischen Abschnittes

21. Nagel (1) nach einem der Ansprüche 1 bis 20, **dadurch gekennzeichnet, dass** er eine Verriegelungsschraube (20) mit einem Schaft (21) und einem Aussengewinde (22) umfasst, wobei für den Durchmesser "d" des Aussengewindes (22) a > d < b gilt, und d vorzugsweise mindestens 5 % kleiner ist als die kleinere der beiden Dimensionen a, b.

22. Nagel (1) nach einem der Ansprüche 1 bis 21, **dadurch gekennzeichnet, dass** der Einsatz (10) aus einem Stift (17) mit konischem erweitertem Kopf (18) besteht.

23. Nagel (1) nach einem der Ansprüche 1 bis 22, **dadurch gekennzeichnet, dass** der Einsatz (10) aus einem zentral durchbohrten Stift (19) mit mehreren peripher angeordneten, konischen Erweiterungen (20) besteht, welche in entsprechende Kavitäten (21), respektive in der Längsbohrung (23) des Nagels (1) im Bereich der Querbohrung (3) im Form eines Klickverschlusses eingreifen können.

## Claims

1. Surgical nail (1), in particular intramedullary nail, comprising a central axis (2) and at least one transverse bore (3) having the cross-sectional profile F and transverse axis (4) running across the central axis (2), whereby the cross-sectional profile F has a maximum length a in the direction of the central axis and a maximum width b perpendicular thereto, and the intramedullary nail has a diameter D in the area of the transverse bore (3), and comprising an insert (10) with a longitudinal axis (13) and a longitudinal slot (15) and which is insertable into the transverse bore (3),
**characterized in that**
the length of the insert (10) coincides with the length of the transverse bore (3).

2. Nail (1) according to Claim 1, **characterized in that** the insert (10) is provided at least partially with an outside thread (16).

3. Nail (1) according to Claim 1 or 2, **characterized in that** the longitudinal slot (15) runs continuously over the entire length of the insert (10).

4. Nail (1) according to any one of Claims 1 through 3, **characterized in that** the insert (10) has several longitudinal slots (15) that do not run over the entire length of the insert (10).

5. Nail (1) according to Claim 4, **characterized in that** the longitudinal slots (15) are arranged with an offset on the circumference of the insert (10).

6. Nail (1) according to Claim 4 or 5, **characterized in that** the longitudinal slots (15) are arranged axially one above the other.

7. Nail (1) according to any one of Claims 1 through 6, **characterized in that** it is made of a material M having the tensile strength F_{z}, the compressive strength F_{d}, the density ρ₂ and the modulus of elasticity E, and the insert (10) is made of a material m which has a lower modulus of elasticity e < E than the material M.

8. Nail (1) according to any one of Claims 1 through 7, **characterized in that** the insert (10) has a bore (14) that is coaxial with its longitudinal axis (13).

9. Nail (1) according to Claim 8, **characterized in that** the longitudinal slot (15) communicates with the coaxial bore (14).

10. Nail (1) according to any one of Claims 1 through 9, **characterized in that** the modulus of elasticity "e" of the insert is e < 0.8E. preferably e < 0.7E.

11. Nail (1) according to Claim 10, **characterized in that** the modulus of elasticity "e" of the insert is e < 0.7E.

12. Nail (1) according to any one of Claims 1 through 11, **characterized in that** the transverse bore (3) is designed to be smooth.

13. Nail (1) according to any one of Claims 1 through 12, **characterized in that** the transverse bore (3) is without a thread.

14. Nail (1) according to any one of Claims 1 through 13, **characterized in that** the material m is a biocompatible plastic, preferably a polyethylene or a high-molecular-weight polyethylene (HMWPE).

15. Nail (1) according to any one of Claims 1 through 14, **characterized in that** the plastic is a bioabsorbable polymer or copolymer, preferably a polylactide.

16. Nail (1) according to any one of Claims 1 through 15, **characterized in that** the transverse bore (3) is a circular bore, whereby the cross-sectional profile F has the maximum lengths a = b.

17. Nail (1) according to any one of Claims 1 through 15, **characterized in that** the transverse bore (3) is designed as an elongated hole, whereby the cross-sectional profile F has the maximum lengths a > b.

18. Nail (1) according to any one of Claims 1 through 17, **characterized in that** the material m has a lower density ρ₁ than the material M which has the density ρ₂, whereby preferably ρ₁ < 0.8 ρ₂.

19. Nail (1) according to any one of Claims 1 through 18, **characterized in that** it comprises a locking screw (20) or a locking pin insertable into the transverse bore (3) through the insert (10), the shaft (21) thereof having a diameter "d" which obeys the conditions a > d < b.

20. Nail (1) according to any one of Claims 1 through 19, **characterized in that** the transverse bore (3) widens toward the surface of the nail (1), preferably in the form of a conical section.

21. Nail (1) according to any one of Claims 1 through 20, **characterized in that** it comprises a locking screw (20) having a shaft (21) and an outside thread (22) whereby for the diameter "d" of the outside thread (22) it holds that a > d < b, and d is preferably at least 5% smaller than the smaller of the two dimensions a, b.

22. Nail (1) according to any one of Claims 1 through 21, **characterized in that** the insert (10) consists of a pin (17) with a conically enlarged head (18).

23. Nail (1) according to any one of Claims 1 through 22, **characterized in that** the insert (10) consists of a pin (19) having a central bore through it with several conical enlargements (20) arranged on the periphery and which are able to engage in corresponding cavities (21) and/or in the longitudinal bore (23) in the nail (1) in the area of the transverse bore (3) in the form of a click closure.

## Revendications

1. Clou chirurgical (1), en particulier clou intramédullaire comprenant un axe central (2) et au moins un alésage transversal (3) s'étendant transversalement à l'axe central (2) ayant un profil de section transversale F et un axe transversal (4), dans lequel le profil de section transversale F, dans le sens de l'axe central, présente une longueur maximale a et, perpendiculairement à celui-ci, une largeur maximale b, et le clou intramédullaire présente, dans la zone de l'alésage transversal (3), un diamètre D, et comprenant un insert (10), ayant un axe longitudinal (13) et une fente longitudinale (15), qui peut être introduit dans l'alésage transversal (3),
**caractérisé en ce que**
la longueur de l'insert (10) correspond à la longueur de l'alésage transversal (3).

2. Clou (1) selon la revendication 1, **caractérisé en ce que** l'insert (10) est pourvu au moins en partie d'un filetage (16).

3. Clou (1) selon la revendication 1 ou 2, **caractérisé en ce que** la fente longitudinale (15) s'étend sur toute la longueur de l'insert (10).

4. Clou (1) selon l'une quelconque des revendications 1 à 3, **caractérisé en ce que** l'insert (10) présente plusieurs fentes longitudinales (15) qui ne s'étendent pas sur toute la longueur de l'insert (10).

5. Clou (1) selon la revendication 4, **caractérisé en ce que** les fentes longitudinales (15) sont disposées de manière décalée sur la périphérie de l'insert (10).

6. Clou (1) selon la revendication 4 ou 5, **caractérisé en ce que** les fentes longitudinales (15) sont superposées axialement.

7. Clou (1) selon l'une quelconque des revendications 1 à 6, **caractérisé en ce qu'**il se compose d'un matériau M ayant une résistance à la traction F_{z}, une résistance à la compression F_{d}, une masse volumique ρ₂ et un module d'élasticité E, et **en ce que** l'insert (10) se compose d'un matériau m qui présente un module d'élasticité inférieur e < E à celui du matériau M.

8. Clou (1) selon l'une quelconque des revendications 1 à 7, **caractérisé en ce que** l'insert (10) présente un alésage (14) coaxial à son axe longitudinal (13).

9. Clou (1) selon la revendication 8, **caractérisé en ce que** la fente longitudinale (15) communique avec l'alésage coaxial (14).

10. Clou (1) selon l'une quelconque des revendications 1 à 9, **caractérisé en ce que** le module d'élasticité "e" de l'insert est e < 0,8 E.

11. Clou (1) selon la revendication 10, **caractérisé en ce que** le module d'élasticité "e" de l'insert est e < 0,7 E.

12. Clou (1) selon l'une quelconque des revendications 1 à 11, **caractérisé en ce que** l'alésage transversal (3) est réalisé sous une forme lisse.

13. Clou (1) selon l'une quelconque des revendications 1 à 12, **caractérisé en ce que** l'alésage transversal (3) n'est pas taraudé.

14. Clou (1) selon l'une quelconque des revendications 1 à 13, **caractérisé en ce que** le matériau m est une matière plastique biocompatible de préférence un polyéthylène ou un polyéthylène de masse moléculaire élevée (HMWPE).

15. Clou (1) selon l'une quelconque des revendications 1 à 14, **caractérisé en ce que** la matière plastique est un polymère ou copolymère biorésorbable, de préférence un polylactide.

16. Clou (1) selon l'une quelconque des revendications 1 à 15, **caractérisé en ce que** l'alésage transversal (3) est un alésage circulaire, dans lequel le profil de section transversale F présente les longueurs maximales a = b.

17. Clou (1) selon l'une quelconque des revendications 1 à 15, **caractérisé en ce que** l'alésage transversal (3) est réalisé en tant qu'alésage oblong, dans lequel le profil de section transversale F présente les longueurs maximales a > b.

18. Clou (1) selon l'une quelconque des revendications 1 à 17, **caractérisé en ce que** le matériau m présente une densité ρ₁ inférieure à celle du matériau M ayant la densité ρ₂, dans lequel, de préférence, ρ₁ < 0,8 ρ₂.

19. Clou (1) selon l'une quelconque des revendications 1 à 18, **caractérisé en ce qu'**il comprend une vis de verrouillage (20) ou un boulon de verrouillage, pouvant être inséré dans l'alésage transversal (3) à travers l'insert (10), dont la tige (21) présente un diamètre "d" qui remplit les conditions a > d < b.

20. Clou (1) selon l'une quelconque des revendications 1 à 19, **caractérisé en ce que** l'alésage transversal (3) s'élargit vers la surface du clou (1), de préférence sous la forme d'un segment conique.

21. Clou (1) selon l'une quelconque des revendications 1 à 20, **caractérisé en ce qu'**il comprend une vis de verrouillage (20) ayant une tige (21) et un filetage (22), dans lequel la relation a > d < b s'applique au diamètre "d" du filetage (22) et d est de préférence inférieur d'au moins 5% par rapport à la plus petite des deux dimensions a, b.

22. Clou (1) selon l'une quelconque des revendications 1 à 21, **caractérisé en ce que** l'insert (10) se compose d'une tige (17) présentant une tête élargie conique (18).

23. Clou (1) selon l'une quelconque des revendications 1 à 22, **caractérisé en ce que** l'insert (10) est formé par une tige transpercée au centre (19) présentant plusieurs élargissements coniques périphériques (20) qui peuvent s'engager à la manière d'un verrouillage par encliquetage dans des cavités correspondantes (21) ainsi que dans l'alésage longitudinal (23) du clou (1) dans la zone de l'alésage transversal (3).
